Europäisches Patentamt

(19) European Patent Office

Office européen des brevets

(11) Publication number: **0 052 879**
**B1**

(12) EUROPEAN PATENT SPECIFICATION

(45) Date of publication of patent specification: 25.09.85

(51) Int. Cl.⁴: **A 61 N 1/36**, H 01 R 13/52

(21) Application number: 81109839.1

(22) Date of filing: 23.11.81

(54) Bipolar coaxial connector with inner sealing grommet.

(30) Priority: 26.11.80 US 210657

(43) Date of publication of application:
02.06.82 Bulletin 82/22

(45) Publication of the grant of the patent:
25.09.85 Bulletin 85/39

(84) Designated Contracting States:
DE FR GB IT NL

(56) References cited:
US-A-3 158 379
US-A-3 855 568
US-A-4 072 154
US-A-4 144 891

(73) Proprietor: MEDTRONIC, INC.
3055 Old Highway Eight
Minneapolis Minnesota 55440 (US)

(72) Inventor: Rose, Maria M.
3105 165th Lane N.E.
Ham Lake Minnesota 55303 (US)

(74) Representative: Schwan, Gerhard, Dipl.-Ing.
Elfenstrasse 32
D-8000 München 83 (DE)

Courier Press, Leamington Spa, England.

## Description

The present invention relates to a coaxial connector for implantable medical devices, and more specifically relates to means for sealing the connection between an implantable lead and an implantable pulse generator or other electrical device.

Cardiac pacers and other similar implantable medical devices contain two basic elements. The first element is an implantable pulse generator which contains the energy source and electronics for supplying the desired stimulus. The second major element is the lead which conveys the electrical stimulating pulse to the desired tissue to be stimulated. To permit convenient implantation and replacement, and for other medical reasons, the two major elements must be connected via a separable connection. Because of the necessity to establish a reliable electrical connection, seal the pulse generator and implantable lead from the ingress of body fluids, and provide rapid connection and disconnection, considerable effort has been expended to optimize connector design. An early connector is known from US—A—3 416 533. This connector uses a number of the techniques common in the electrical arts for nonimplantable devices.

US—A—3,760,332 teaches a newer connector for bipolar use. This connector uses two separate and parallel connector pins inserted in a single housing. Sealing of the connector is accomplished through the use of multiple sealing rings attached to the implantable lead portion of the connector which seal the connection against the pulse generator housing.

Later implantable connectors are described in US—A—4 072 154, in US—A—4 112 953, in US—A—4 142 532 and in US—A—4 144 891. These references show common sealing methods. According to US—A—4 112 953, for example, sealing is achieved by suture material tightened over a boot during the implant procedure. Whereas this technique may be effective and relatively inexpensive, it is time-consuming and must be accomplished during the implant procedure. A second prevalent method is to use a material on the proximal end of the implantable lead which is substantially more pliant than the relatively rigid lumen of the pulse generator connector housing as shown in US—A—4 142 532. The seal is thus created by compression of the relatively pliant material at the proximal end of the implantable lead. Oftentimes this technique is enhanced through the use of one or more sealing rings which are connected to the proximal end of the implantable lead (US—A—3 760 332, US—A—7 072 154, US—A—4 144 891).

A common problem with these sealing techniques is that, they either require substantial operating room time to create the proper seal, or require a compressible material at the proximal end of the implantable lead. This causes an increase in the cross-sectional area of the proximal end of the implantable lead. A further problem noted in the cited references and considered prominent in the art is that the requirement for more than one electrical connection must be met with multiple parallel connector pins. It is felt that this design problem is primarily related to the difficulty in establishing an acceptable seal with a minimal cross-sectional area of the proximal end of the lead and with ease of initial implant and replacement of the pulse generator element.

A coaxial connector for coupling a proximal end of an implantable lead to an implantable pulse generator comprises: a connector housing of an insulating material attached to said implantable pulse generator, the housing having a lumen for the insertion of said proximal end of said implantable lead;

an exposed electrically conductive surface adjacent said proximal end of said implantable lead;

an electrically insulating surface adjacent said proximal end of said implantable lead;

a terminal block attached to said connector housing within said lumen to electrically engage said electrically conductive surface when said proximal end of said implantable lead in inserted into said lumen of said connector housing; and

a plurality of sealing elements, in conformity with the invention is characterized in that a plurality of exposed electrically conductive surfaces and a plurality of electrically insulating surfaces alternating with said conductive surfaces are provided adjacent said proximal end of said lead;

a plurality of terminal blocks are attached to said connector housing, each of said terminal blocks engaging a different one of said plurality of electrically conductive surfaces; and

said sealing elements comprise sealing grommets which are attached to said connecting housing within said lumen whereby each of said plurality of sealing grommets sealingly engages a different one of said plurality of electrically insulating surfaces.

Key to the present invention is the moving of the sealing mechanism from the proximal end of the lead to a position within the lumen of the pulse generator connector housing. Movement of the sealing mechanism in this fashion permits a substantial reduction in the cross-sectional area of the proximal end of the implantable lead. By so reducing this cross-sectional area, connectors having multiple conductors may be easily arranged in coaxial fashion.

The connector housing of the implantable pulse generator is a single molded element of a transparent rigid material. A single lumen is present within the connector housing for the insertion of the proximal end of the implantable lead. A number of apertures are present which are orthogonal to the lumen. Inserted into these apertures are the sealing grommets and connector blocks. Each sealing grommet is a separately molded element having pliant sealing rings within its inner lumen. Each connector block is an electrically conducting structure also having an inner lumen. The sealing grommets and terminal blocks are inserted within the orthogonal apertures of the connector housing in such a fashion that the inner lumens of the sealing grommets and the connector blocks are in line with the lumen of the connector housing. Sealing grommets and terminal blocks are arranged within the orthogonal apertures of the connector housing alternately. That is, a sealing grommet is located within the most distal orthogonal aperture. A terminal block is located within the second most distal aperture. A sealing grommet is located within the third most distal aperture and so forth.

The proximal end of the implantable lead has connector surfaces and insulated surfaces which are also alternated. The most proximal surface of the implantable lead is a conductor surface. The next most proximal surface is an insulator. The next most proximal surface is a conductor and so forth. On insertion of the proximal end of the implantable lead into a lumen of the connector housing each conductor surface of the implantable lead is aligned with a terminal block and each insulating surface of the implantable lead is aligned with a corresponding sealing grommet. It can be seen that this technique is applicable to sealable coaxial connectors having any number of separate and mutually insulated conductors.

One way of carrying out the invention is described in detail below with reference to the drawings which illustrate only one specific embodiment,

Fig. 1 is a side view of a pulse generator and implantable lead before insertion employing the present invention.

Fig. 2 is a top sectional view of the molded connector housing.

Fig. 3 is a perspective view of one of the sealing grommets.

Fig. 4 is a bottom view of the connector housing showing the othogonal apertures.

The preferred embodiment of the present invention utilizes a connector having two conductors. This is because the preferred embodiment employs a bipolar pacing lead. However, the present invention likewise is applicable to connectors having more than two conductors.

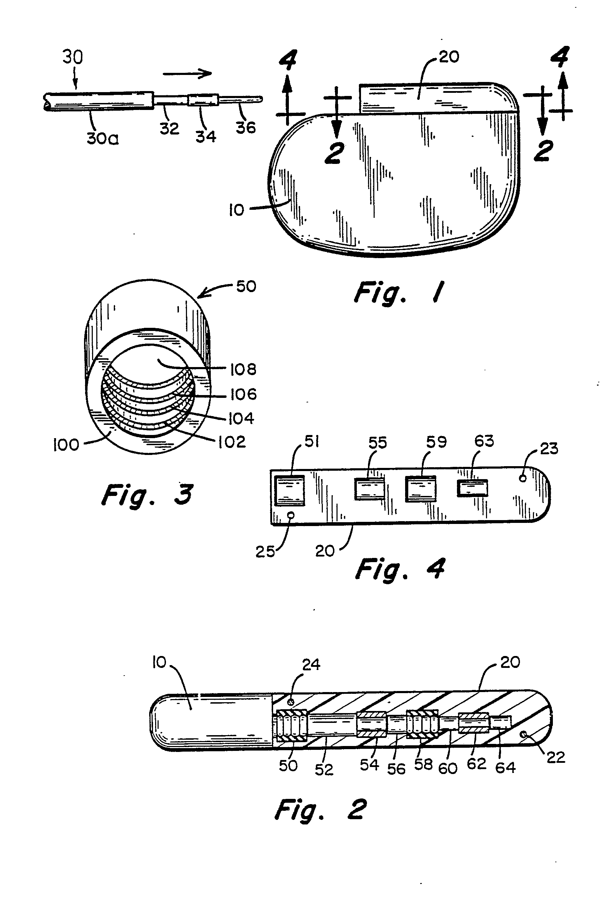

Fig. 1 shows a side of a pacing lead and implantable pulse generator employing the present invention. Pulse generator body 10 is attached to connector housing 20 using techniques known from US—A—4 142 532.

Lead 30 has a proximal end and a distal end having two electrodes. The distal end is not shown for clarity. The main body of lead 30 is covered by an insulating sheath 30a of a material which is substantially inert to body fluids and is sufficiently flexible. The materials commonly used are silicon rubber or urethane. Proximal end of lead 30 has two coaxial conductor areas 32 and 36. Each conductor (i.e. conductors 32 and 36) is connected to a different one of the distal electrodes (not shown). Conductor 32 and conductor 36 are separated by insulating material 34 as shown. The proximal end of lead 30 is inserted into connector housing 20 in the direction of the arrow as shown. Connector housing 20 has a longitudinal lumen into which lead 30 is inserted.

Fig. 2 is a top-sectional view of the connector housing. Connector housing 20 is attached to pulse generator 10 via mounting pins 22 and 24. The longitudinal lumen 52, 56, 60, 64 within connector housing 20 is of varying diameters as shown. By referring back to Fig. 1, it can be seen that the lead diameter of the main body of the lead 30 is greater, for example, than any portion proximal thereto. It can also be seen that conductor 36, for example, has a smaller diameter than conductor 32. Referring again to Fig. 2, it can be appreciated that lead 30 is inserted into the lumen within connector housing 20. Upon being inserted conductor 36 is lodged within terminal block 62 and within the narrower portion 64 of the lumen. Sealing grommet 58 frictionally engages insulating portion 34 of the lead. Conductor 32 is frictionally engaged by terminal block 54 and the outer sheath 30 is frictionally engaged by sealing grommet 50.

Sealing grommets 50 and 58 frictionally engage insulated portions of the proximal end of the lead whereas terminal blocks 54 and 62 frictionally engage the conductor portions. It is further significant that sealing grommets 50 and 58 alternate in the longitudinal direction with terminal block 54 and 62. This permits an effective seal to be made between each of the connector elements of the proximal end of lead 30 causing a reduced leakage current.

As explained above, any number of sealing grommets and terminal blocks may be used in coaxial relationship and in alternating fashion as shown to achieve a connector having the requisite number of conductor elements. It is important however, that they are alternated to separately seal each of the conductor elements and the sealing grommet 50 must be located at the most distal end of the connector assembly, since it is required to seal the first conductor from body fluids. The construction and use of terminal blocks 54 and 62 are known from US—A—4 142 532.

Fig. 3 shows the construction of one of the sealing grommets 50. It is a separately molded item of a material such as silicon rubber or urethane which is substantially pliant to allow compression during insertion of the proximal end of lead 30. The outer body of sealing grommet 50 is sufficiently smooth for insertion into the connector housing 20 and for attachment thereto. Inner lumen 108 is sufficiently large to accept the diameter of that portion of the proximal end of lead 20 which it will encase. Three inner sealing rings are located within inner lumen 108. These sealing rings (i.e. sealing ring 102, sealing ring 104, and sealing ring 106) have an inside diameter smaller than that corresponding diameter of lead 30 to enable sealing rings 102, 104 and 106 to be compressed and thereby frictionally engage the corresponding portion of lead 30.

Fig. 4 is a bottom view of connector housing 20. The connector housing contains apertures 51, 55, 59 and 63. These apertures are essentially rectangular in shape. These apertures extend from the bottom surface of connector housing 20 to the longitudinal lumen within the connector housing 20. Sealing grommet 50 is attached within aperture 51 such that lumen 108 is aligned with the longitudinal lumen of connector housing 20. Similarly, sealing grommet 58 is inserted and attached within aperture 59. This attachment may be provided through suitable adhesives or other common attachment means. Terminal blocks 54 and 62 are similarly inserted into apertures 55 and 63 respectively. Round apertures 23 and 25 are used for attachment of connector housing 20 to pulse generator 10 with mounting pins 22 and 24. See also Fig. 2.

**Claims**

1. A coaxial connector for coupling a proximal end of an implantable lead (30) to an implantable pulse generator (10) comprising:
   a connector housing (20) of an insulating material attached to said implantable pulse generator, the housing having a lumen (52, 56, 60, 64) for the insertion of said proximal end of said implantable lead;
   an exposed electrically conductive surface adjacent said proximal end of said implantable lead;
   an electrically insulating surface adjacent said proximal end of said implantable lead;
   a terminal block attached to said connector housing within said lumen to electrically engage said electrically conductive surface when said proximal end of said implantable lead is inserted into said lumen of said connector housing; and
   a plurality of sealing elements,
   characterized in that
   a plurality of exposed electrically conductive surfaces (32, 36) and a plurality of electrically insulating surfaces (30a, 34) alternating with said conductive surfaces are provided adjacent said proximal end of said lead (30);
   a plurality of terminal blocks (54, 62) are attached to said connector housing (20), each of said terminal blocks engaging a different one of said plurality of electrically conductive surfaces (32, 36); and
   said sealing elements comprise sealing grommets (50, 58) which are attached to said connector housing (20) within said lumen (52, 56, 60, 64) whereby each of said plurality of sealing grommets sealingly engages a different one of said plurality of electrically insulating surfaces (30a, 34).

2. A coaxial connector according to claim 1 wherein each of said plurality of electrically insulating surfaces (30a, 34) is located immediately distal a corresponding one of said plurality of electrically conductive surfaces (32, 36).

3. A coaxial connector according to claim 1 or 2 wherein each of said sealing grommets further comprises at least one sealing ring (102, 104, 106).

4. A coaxial connector according to any one of claims 1 to 3 wherein said connector housing (20) is rigid.

**Revendications**

1. Connecteur coaxial pour connecter une extrémité proximale d'un conducteur implantable (30) à un générateur d'impulsions implantable (10), comportant: un boitier de connecteur (20) d'une matière isolante fixé sur ledit générateur d'impulsions implantable, le boitier comportant un orifice (52, 56, 60, 64) pour l'introduction de ladite extrémité proximale dudit conducteur implantable; une surface conductrice de l'électricité, exposée et voisine de ladite extrémité proximale dudit conducteur implantable; une surface isolante de l'électricité voisine de ladite extrémité proximale dudit conducteur implantable; un bloc de connexion fixé sur ledit boitier de connecteur dans ledit orifice pour engager électriquement ladite surface conductrice de l'électricité quand ladite extrémité proximale dudit conducteur implantable est introduite dans ledit orifice dudit boitier de connecteur, et plusieurs éléments d'étanchéité, caractérisé en ce que plusieurs surfaces conductrices de l'électricité exposées (32, 36) et plusieurs surfaces isolantes de l'électricité (30a, 34) alternées avec lesdites surfaces conductrices sont prévues près de ladite extrémité proximale dudit conducteur 30; plusieurs blocs de connexion (54, 62) étant fixés sur ledit boitier de connecteur (20), chacun desdits blocs de connexion engageant l'une différente desdites surfaces conductrices de l'électricité (32, 36); et lesdits éléments d'étanchéité consistant en des garnitures d'étanchéité (50, 58) qui sont fixées audit boitier de connecteur (20) dans ledit orifice (52, 54, 60, 64) de manière que chacune desdites garnitures d'étanchéité engage de façon étanche l'une différente desdites surfaces isolantes de l'électricité (30a, 34).

2. Connecteur coaxial selon la revendication 1, dans lequel chacune desdites surfaces isolantes de l'électricité (30a, 34) est située dans une partie immédiatement distale de l'une correspondante desdites surfaces conductrices de l'électricité (32, 36).

3. Connecteur coaxial selon la revendication 1 ou 2, dans lequel chacune desdites garnitures d'étanchéité comporte également au moins une bague d'étanchéité (102, 104, 106).

4. Connecteur coaxial selon l'une quelconque des revendications 1 à 3, dans lequel ledit boitier de connecteur (20) est rigide.

**Patentansprüche**

1. Koaxial-Steckverbindung zum Koppeln eines proximalen Endes einer implantierbaren Leitung (30) mit einem implantierbaren Impulsgenerator (10), versehen mit:
   einem an dem implantierbaren Impulsgenerator angebrachten Steckverbindungsgehäuse (20) aus einem isolierenden Werkstoff, wobei das Gehäuse einen Hohlraum (52, 56, 60, 64) zum Einführen des proximalen Endes der implantierbaren Leitung aufweist;

einer freiliegenden, elektrisch leitenden Fläche benachbart dem proximalen Ende der implantierbaren Leitung;

einer elektrisch isolierenden Fläche benachbart dem proximalen Ende der implantierbaren Leitung;

einem an dem Steckverbindungsgehäuse innerhalb des Hohlraums angebrachten Anschlußblock, der mit der elektrisch leitenden Fläche elektrisch in Eingriff kommt, wenn das proximale Ende der implantierbaren Leitung in den Hohlraum des Steckverbindungsgehäuses eingeführt wird; und

einer Mehrzahl von Dichtungselementen,

dadurch gekennzeichnet, daß

benachbart dem proximalen Ende der Leitung (30) eine Mehrzahl von freiliegenden, elektrisch leitenden Flächen (32, 36) und eine Mehrzahl von mit diesen leitenden Flächen abwechselnden, elektrisch isolierenden Flächen (30a, 34) vorgesehen sind;

an dem Steckverbindungsgehäuse (20) eine Mehrzahl von Anschlußblöcken (54, 62) angebracht ist, von denen jeder mit einer anderen der Mehrzahl von elektrisch leitenden Flächen (32, 36) in Eingriff steht; und

die Dichtungselemente dichtende Durchführungen (50, 58) aufweisen, die an dem Steckverbindungsgehäuse (20) innerhalb des Hohlraums (52, 56, 60, 64) angebracht sind, wobei jede der Mehrzahl von dichtenden Durchführungen mit einer anderen der Mehrzahl von elektrisch isolierenden Flächen (30a, 34) in dichtendem Eingriff steht.

2. Koxial-Steckverbindung nach Anspruch 1, wobei jede der Mehrzahl von elektrisch isolierenden Flächen (30a, 34) unmittelbar distal von einer entsprechenden der Mahrzahl von elektrisch leitenden Flächen (32, 36) angeordnet ist.

3. Koaxial-Steckverbindung nach Anspruch 1 oder 2, wobei jede der dichtenden Durchführungen ferner mindestens einen Dichtring (102, 104, 106) aufweist.

4. Koaxial-Steckverbindung nach einem der Ansprüche 1 bis 3, wobei das Steckverbindungsgehäuse (20) starr ist.

Fig. 1

Fig. 3

Fig. 4

Fig. 2